# EUROPEAN PATENT SPECIFICATION

(11) **EP 1 698 646 B1**
(45) Date of publication and mention of the grant of the patent: **21.11.2007**
(21) Application number: 05004802.4
(22) Date of filing: 04.03.2005
(51) Int. Cl.: C08F 222/10, C08F 236/00

(54) **Photocurable composition and photocurable compound**
Lichthärtbare Zusammensetzung und lichthärtbare Verbindung
Composition photodurcissable et composé photodurcissable

(43) Date of publication of application: 06.09.2006
(73) Proprietor: DAINIPPON INK AND CHEMICALS, INC., Itabashi-ku Tokyo (JP)
(72) Inventor: Ferbitz, Jens Dr., 10439 Berlin (DE); Lachowicz, Artur Dr., 13467 Berlin (DE); Gaudl, Kai-Uwe Dr., 16540 Hohen Neuendorf (DE)
(74) Representative: Albrecht, Thomas

(56) References cited:
- EP-A- 1 342 737
- EP-A- 1 342 738
- US-A- 6 025 410

## Description

### BACKGROUND OF THE INVENTION

### FIELD OF THE INVENTION

The present invention relates to a photocurable composition, and a new curable compound, which is usable as a constituent of the composition. More specifically, the composition of the present invention is usable for coatings, or molded articles which are curable with UV light to sunlight without any use of photoinitiators.

### DESCRIPTION OF RELATED ART

A photocurable composition containing a resin having plural acryloyl groups, which can be cured by irradiation with UV (ultraviolet) light or sunlight, is widely used in fields of coatings, printing inks or molded articles.

For this curing system, in general, a photoinitiator had been used together with the acryloyl group containing resin in the abovementioned fields. Namely, the photoinitiator can generate free-radicals easily under photoirradiation and initiates free-radical polymerization of the acryloyl groups, which then leads to a hardening of the resin.

However, the above-mentioned radiation-hardening systems containing photoinitiators had given rise to several practical problems. For example, unreacted photoinitiators as well as their cleavage products usually remained in the coating , migrated to the surface , and affected adjacent products. Particularly, in the foodstuff packaging industry, migration was quite serious problem. That is, the migrated unreacted photoinitiators and their cleavage products led to contamination of the foodstuffs themselves.

Furthermore, residues of photoinitiators such as benzophenone, or volatile cleavage products such as benzaldehyde, that are formed throughout radical formation by homolytic cleavage of α-cleavable compounds, in addition led to the formation of odor that adversely affected the quality of the hardened products.

Additionally some of low-molecular weight cleavage products are toxic and often caused a serious problem for the consumer especially in foodstuff packaging.

Another serious problem that has been caused by photoinitiators was yellowing, which was caused by amine based coinitiators or side-reaction products of aromatic groups within the structure of the initiator,.

On account of the aforementioned disadvantages, the need has arisen in the technology to provide a photocurable acryloyl group containing resin system that can harden without use of photoinitiators.

As a means for overcoming these problems, there have been proposed a photoinitiator-free oligomeric composition having acryloyl groups. For example, United State Patent No. 6025410 discloses liquid oligomeric compositions produced by Michael Addition reaction of acetoacetates with acrylates, that can be polymerized under UV light in the absence of photoinitiators. Furthermore, EP-A1-1342738 discloses a further improved self-curable liquid oligomeric composition, which is also given by Michael addition reaction of acetoacetates with acrylates. That is, the composition can give a tack-free cured product by introducing tertiary carbon atom structures into an acryloyl group containing resin.

The liquid oligomeric compositions disclosed in these documents, however, had a problem in that photo-reactivity was not enough, thereby had caused tacky surfaces of the cured films at high curing-speed due to severe oxygen inhibition.

### SUMMARY OF THE INVENTION

It is therefore an object of the present invention to provide an aromatic free photocurable composition, which has higher curability without any photoinitiators, and thereby shows reduced oxygen inhibition and gives products with good hardness and solvent resistance. Another object is to provide a compound, which can give the abovementioned higher self-curability to the composition, and can thereby give cured products having excellent hardness and as solvent resistance.

Unexpectedly, we have now found that, with respect to chemical structure of acryloyl group containing resin, by introducing β (beta)-diketone structure having specific substituent group at α (alpha) -position to both carbonyl groups, the photoreactivity of the composition can be extremely enhanced.

Accordingly, the present invention provides, a photocurable composition containing a resin (A) having an acryloyl group and a chemical structure element represented by the following formula I, 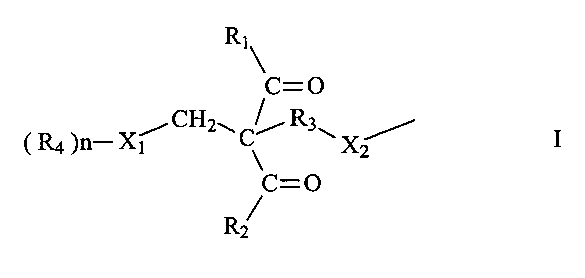 wherein R1 and R2 independently denote an alkyl or cycloalkyl group having a carbon number of 1 to 6, X1 denotes carbonyl group or cyano group, R4 denotes an alkyl or alkoxy group having carbon number of 1 to 8, n denotes 1 when X1 is carbonyl, and 0 when X1 is cyano group, R3 denotes an alkylene group having a carbon number of 1 to 3, and X2 denotes an electron attracting group.

The present invention also provides a photocurable compound, which is represented by the following formula III, 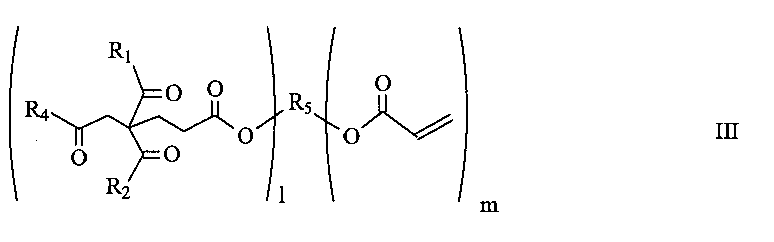 wherein R1 and R2 independently denote an alkyl or cycloalkyl group having a carbon number of 1 to 6, R4 denotes an alkyl or alkoxy group having carbon number of 1 to 8, R5 denotes a polyvalent aliphatic hydro-carbon group having a carbon number of 2 to 8, 1 means an integer of 1 or 2, and m means an integer of 1 to 3.

According to the present invention, a photocurable composition can be provided, which exhibits, by irradiation withUV light or sunlight, higher curability without any photoinitiators, and thereby gives good hardness such as solvent resistance to the cured products.

Furthermore, a photocurable compound usable as a constituent of the composition can be also provided, which gives the aforementioned property to the composition.

Therefore, the composition of the present invention is suitable for coatings, or moldings.

### DETAILED DESCRIPTION OF THE PREFERRED EMBODIMENTS

The embodiments of the present invention will hereinafter be described in detail.

A resin to be incorporated in the photocurable composition of the present invention has an acryloyl group, and a chemical structure element represented by the following formula I.

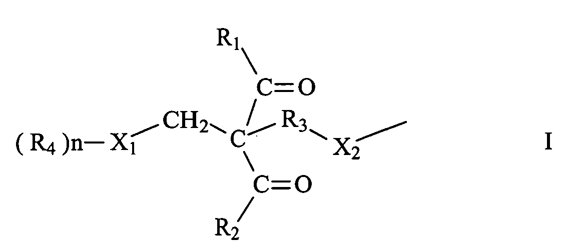

Here, in the formula I, R1 and R2 independently denote an alkyl or cycloalkyl group having a carbon number of 1 to 6, such as methyl, ethyl, n-propyl, i-propyl, n-butyl, i-butyl, t-butyl, and cyclohexyl. In the present invention, from the viewpoints of contribution to the reactivity of radicals towards double-bonds, preferred are methyl and t-butyl.

As aforementioned, X1 denotes carbonyl group or cyano group, R4 denotes an alkyl or alkoxy group having carbon number of 1 to 8, and n denotes 1 when X1 is carbonyl,and 0 when X1 is cyano group. That is, the sensitivity for photo-induced α (alpha)-cleavage between the α (alpha)-carbon atom and R1CO- (or R2CO-) in the formula I can be surprisingly enhanced by bonding the carbonyl group or cyano group to the α (alpha)-carbon atom in the β (beta)-diketone via methylene group. Here, examples of the alkyl or alkoxy group having carbon number of 1 to 8 include methyl, ethyl, n-propyl, i-propyl, n-butyl, i-butyl, t-butyl, n-pentyl, n-hexyl, cyclo-hexyl, heptyl, octyl, methoxy, ethoxy, n-propoxy, i-propoxy, n-butoxy, t-butoxy, n-pentyloxy, n-hexyloxy, cyclo-hexyloxy, heptyloxy, and octyloxy.

Next, R3 denotes an alkylene group having a carbon number of 1 to 4,such as methylene, ethylene, 1,3-propylene, 2,2-propylene, 1,2-dimethylethylene, 2,2-dimethylethylene, and 1,1-dimethylethylene. In the present invention, by selecting R3 so that the distance between the α(alpha)-carbon atom in the β(beta)-diketone and the electrone attracting group (X2) is within a range of a carbon number of 3, the superior photocurability can be attained.

Further, X2 denotes an electron attracting group as aforementioned. Examples of the electron attracting group include a ketone group, an carbonyloxy group, an ether group, carboxyl, a cyano group, a sulfonic acid group, a sulfonyl group, a phosphate group.

Among these structural elements represented in the aforementioned formula I, the following formula II is preferable from the viewpoints of productivity thereof as well as photocurability in the resin. 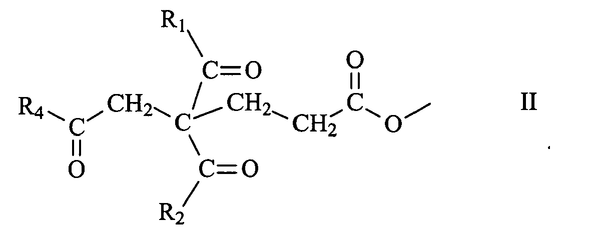 Here, R1, R2, and R4 are defined in formula I.

These resins having an acryloyl group together with a chemical structural element represented by the aforementioned formula I, preferably formula II, can be obtained by standard well-known alkylation, and if necessary, further Michael Addition reaction as follows.

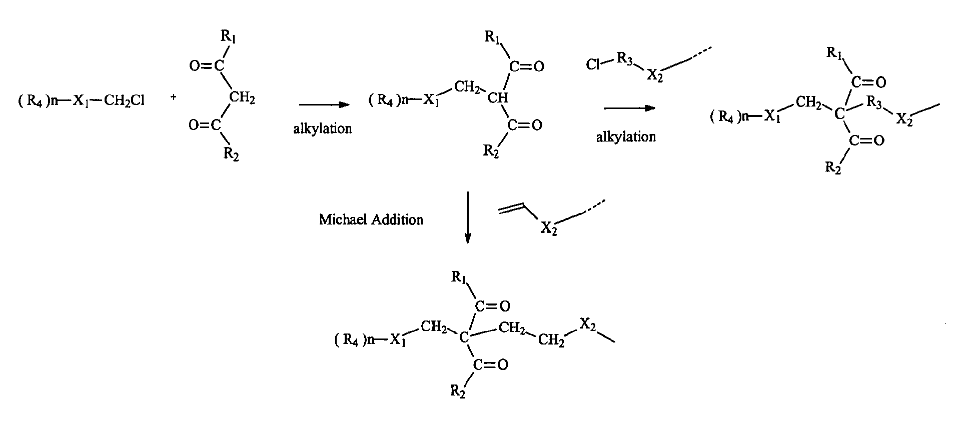

In the abovementioned reaction scheme, the alkylation of β-diketone with halogenated alkyl can be carried out in accordance with aliphatic nucleophilic substitution, that includes a conventional process disclosed by Yoshimura, Saito, Tamura, Tanikaga, Kaj, Bull Chem. Soc. Jpn. 52, p.1716. (1979) or House "Modern Synthetic Reactions", 2nd, ed., pp. 492 - 570, 586 - 595 or Fedorynski, Wojciechowski, Matacz, Makosza, J. Org. Chem. 43, 4682. (1978) (the contents of which are hereby incorporated by reference).

In the case of Michael Addition reaction of β-diketone with acrylates, for example, which can be carried out in accordance with Bergmann, Ginsburg, Pappo, Org. React. 10, p. 179-560 (1959), (the contents of which are hereby incorporated by reference). Examples of catalysts of the Michael addition preferably include amines such as triethyl amine, 1,8-diazabicyclo[5.4.0]undec-7-ne, 1,5-diazabicyclo[4.3.0]non-5-ne, 1,4-diazabicyclo[2.2.2]octan, alkoholates such as sodium methylate, sodium ethylate, potassium tert-butylate, and phosphines such as trioctyl phosphine.

Furthermore, as for sulfoxide structural element, the synthesis thereof can proceed through the Michael addition of vinyl sulfone to β-diketone (Truce, W. E., Wellisch, E.; J. Amer. Chem. Soc. 74, 2881. (1952), the contents of which are hereby incorporated by reference).

As a starting material of the alkylation, the compounds represented by the following formula can be used as above illustrated. Here, Hal denots a halogen atom such as Chlorine atom, and Bromin atom.

(R₄)ₙ-X₁-CH₂-Hal

The examples of the formula include methyl chloroacetate, methyl bromoacetate, ethyl bromoacetate, t-butyl bromoacetate, ethyl chloroacetate, propyl chloroacetate, n-butyl chloroacetate, t-butyl chloroacetate, pentyl chloroacetate, cyclohexyl chloroacetate, heptyl chloroacetate, octyl chloroacetate, chloroacetone, ethyl-chloromethylketone, propyl-chloromethylketone, n-butyl-chloromethylketone , t-butyl-chloromethylketone , pentyl-chloromethylketone , cyclohexyl-chloromethylketone , heptyl-chloromethylketone, octyl-chloromethylketone, and chloromethylcyanide.
Examples of β-diketone are represented by the following formula 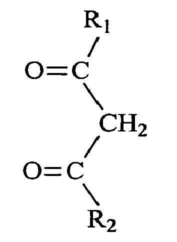 , wherein R1 and R2 are defined in formula 1, include for example; pentane-2,4-dione, hexane-2,4-dione, heptane-2,4-dione, heptane-3,5-dione, octane-2,4-dione, octane-3,5-dione, nonane-2,4-dione, nonane-3,5-dione, nonane-4,6-dione, decane-2,4-dione, decane-3,5-dione, decane-4,6-dione, undecane-2,4-dione, undecane-3,5-dione, undecane-4,6-dione, undecane-5,7-dione, 2,2-dimethyl-hexane-3,5-dion, 2,2,6,6-tetramethyl-heptane-3,5-dione and β-diketone compounds represented by the following formula.

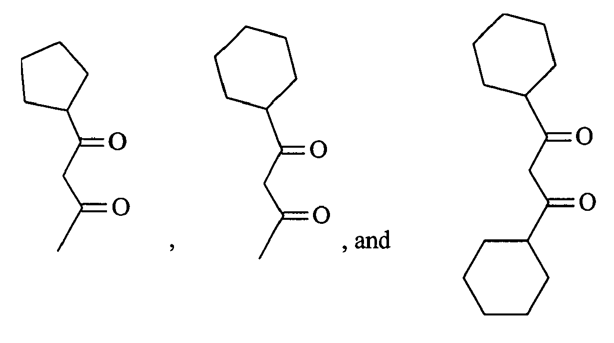

In case of producing the compound (A) via Michael addition reaction, a poly-functional acrylic acid ester can be preferably used in the present invention. Here the poly-functional acrylic acid ester include for example: 1,2-ethanediol diacrylate, 1,3-propanediol diacrylate, 1,4-butanediol diacrylate, 1,6-hexanediol diacrylate, dipropylene glycol diacrylate, neopentyl glycol diacrylate, ethoxylated neopentyl glycol diacrylate, propoxylated neopentyl glycol diacrylate, tripropylene glycol diacrylate, bisphenol A diglycidyl ether diacrylate, ethoxylated bisphenol A diglycidyl ether diacrylate, polyethylene glycol diacrylate, trimethylolpropane triacrylate, ethoxylated trimethylolpropane triacrylate, propoxylated trimethylolpropane triacrylate, propoxylated glycerol triacrylate, tris(2-acryloyloxyethyl) isocyanurate, pentaerythritol triacrylate, ethoxylated pentaerythritol triacrylate, pentaerythritol tetraacrylate, ethoxylated pentaerythritol tetraacrylate, ditrimethylolpropane tetraacrylate,
dipentaerythritol pentaacrylate, dipentaerythritol hexaacrylate, acrylate group-containing oligomers and polymers obtained by reacting polyepoxides with acrylic acid (epoxyacrylates) or obtained by reacting polyester polyols with acrylic acid and/or monomeric alkyl acrylates (polyester acrylates).

Particularly preferable examples include trimethylolpropane triacrylate or pentaerythritol tetraacrylate from the viewpoint of hardness of cured product thereof.

After synthesis process, purification may be carried out in various ways. For example, the product may be passed through an acidic ion exchanger that removes a basic catalyst, or the catalyst can be neutralized by addition of acids and then precipitated and filtered off. Combined anion exchangers and cation exchangers are suitable for removing salt-type catalysts.

Thus, obtained products obtained by the aforementioned process are liquid, with viscosities of 200 mPa·s at 25°C to_10Pa·s at 25°C. Preferable viscosity is in the range between 500 and 2000 mPa·s at 25°C.

In the present invention, a molecular weight (Mn) of the aforementioned resin (A) is preferably within a range of 300 to 20,000,from the viewpoint of viscosity.

Further, a preferred concentration of the total amount of the acyl group A1 and A2 which are represented by following formula, 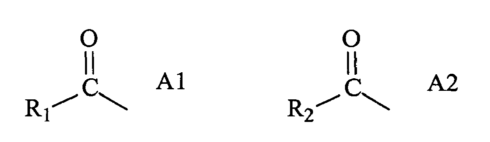 wherein R1 and R2 denotes an alkyl group having a carbon number of 1 to 6, is between 1·10⁻⁵ mole per gram and 1·10⁻² mole per gram, based on the total amount of the composition from the viewpoints of curing speed, particularly between 1·10⁻⁴ mole per gram and 1.10⁻³ mole per gram.

In the present invention, it is safe to say that all of compounds, which are obtainable from the abovementioned starting materials, can be used as a resin (A). However, among them, preferable are the compounds having 1 to 3 acryloy groups in light of hardness.

Examples of mono-functional acryloyl group containing compounds, include the compounds represented by the following chemical formula.

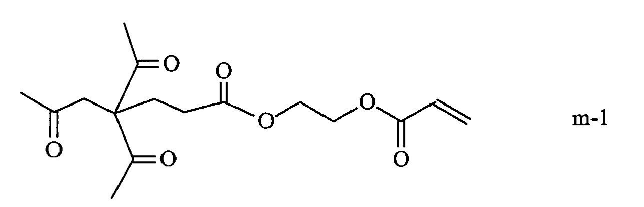

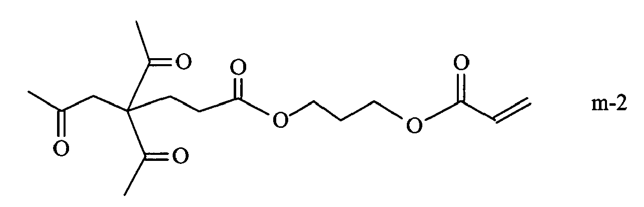

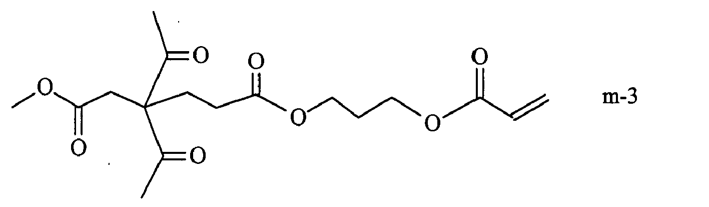

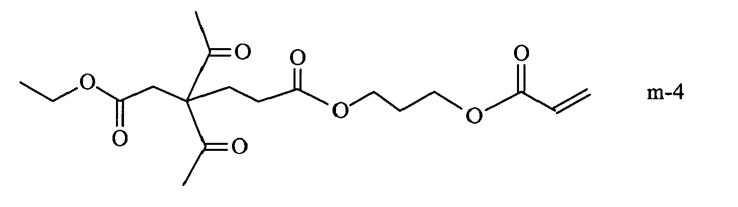

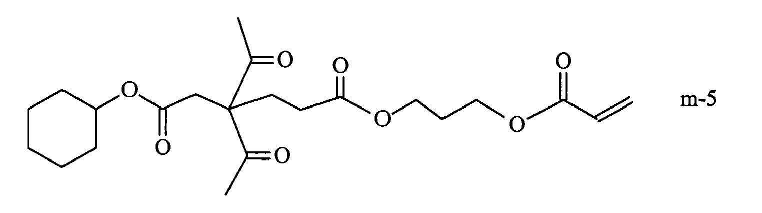

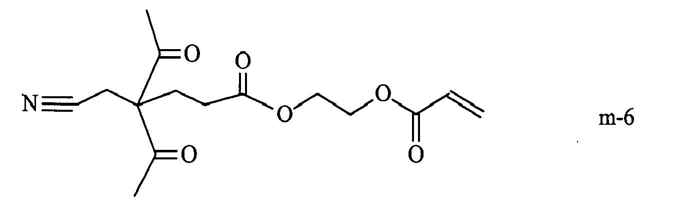

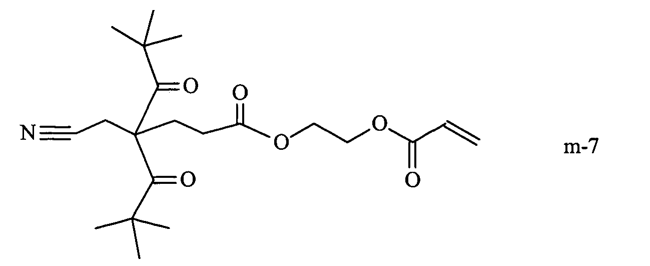

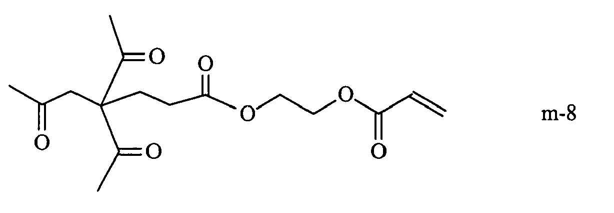

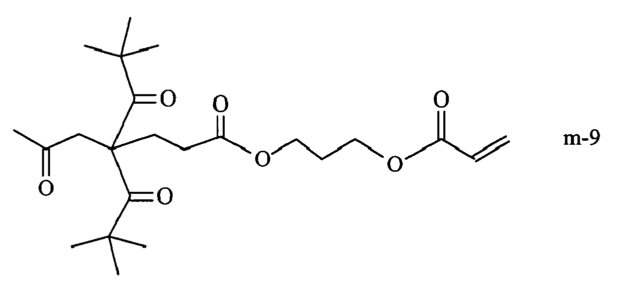

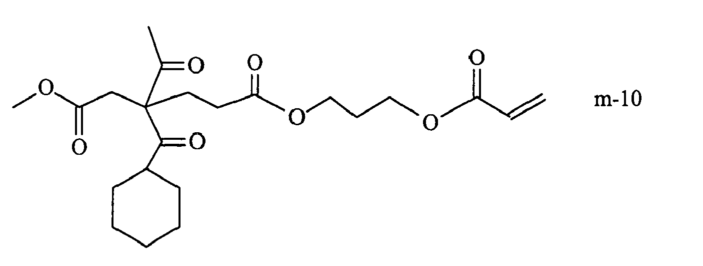

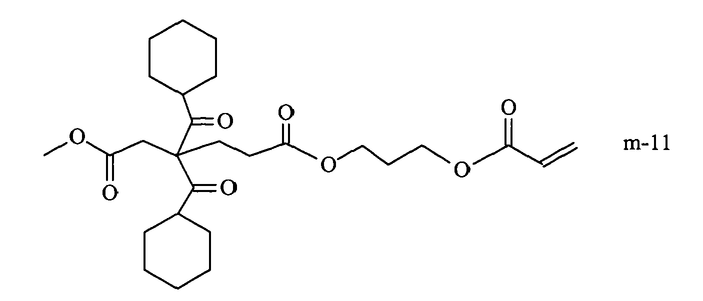

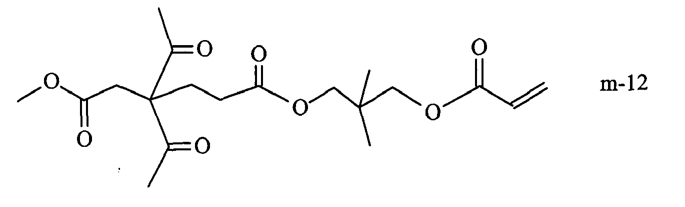

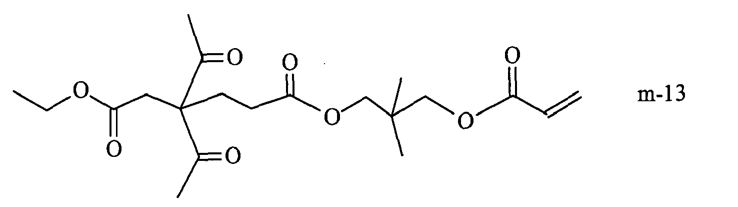

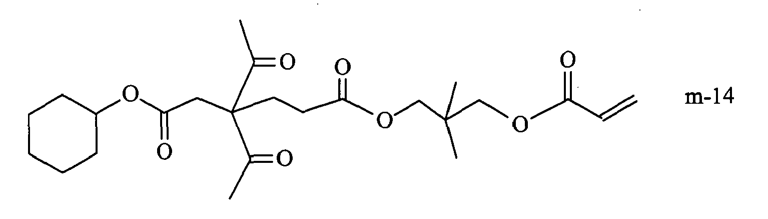

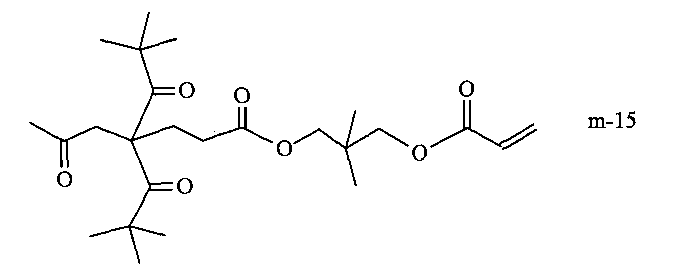

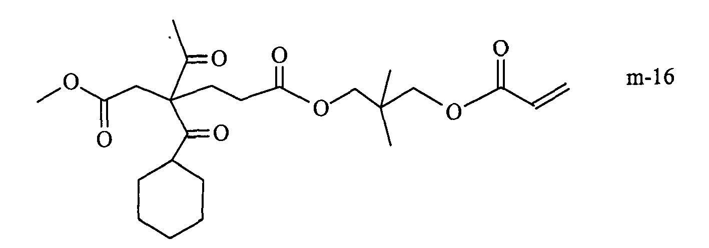

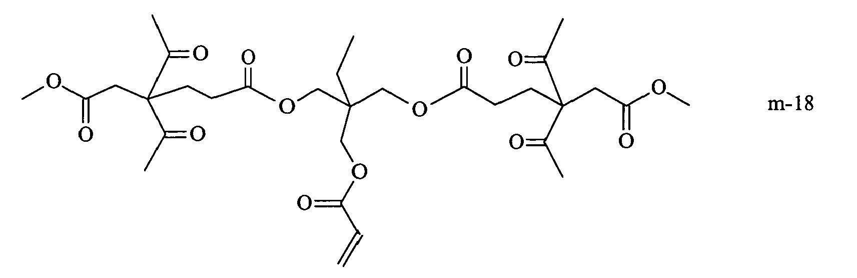

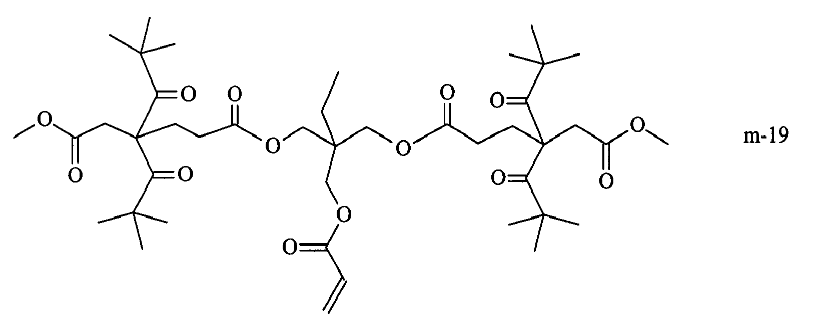

Examples of di-functional acryloyl group containing compounds, include the compounds represented by the following chemical formula.

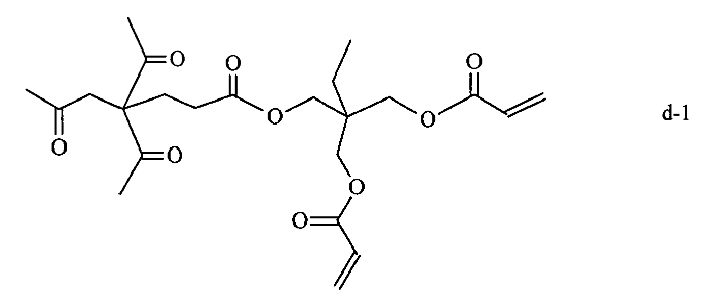

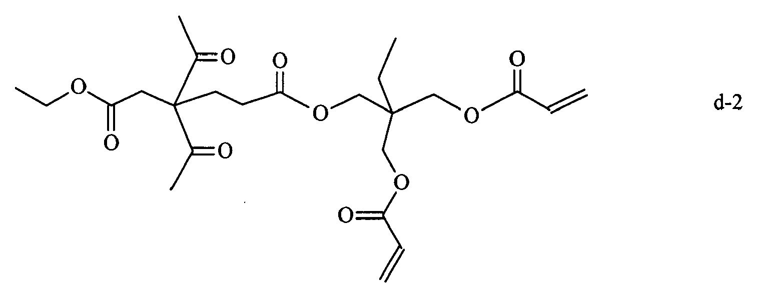

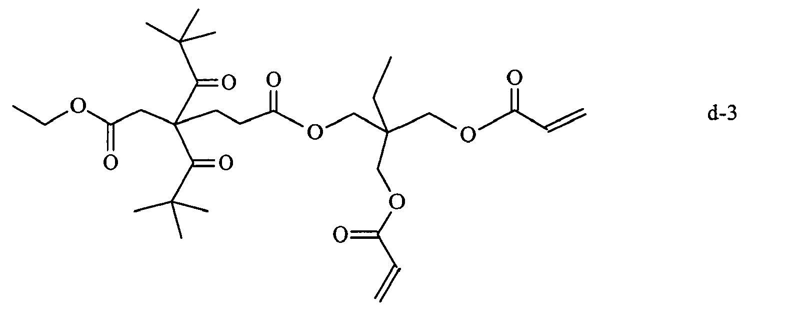

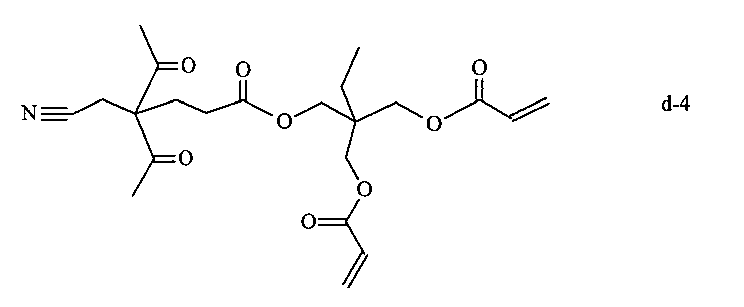

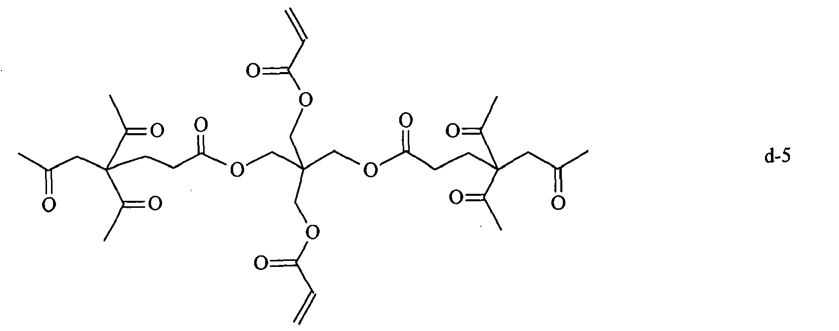

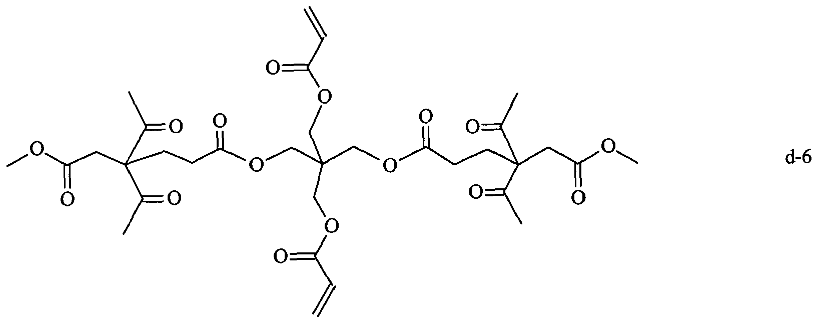

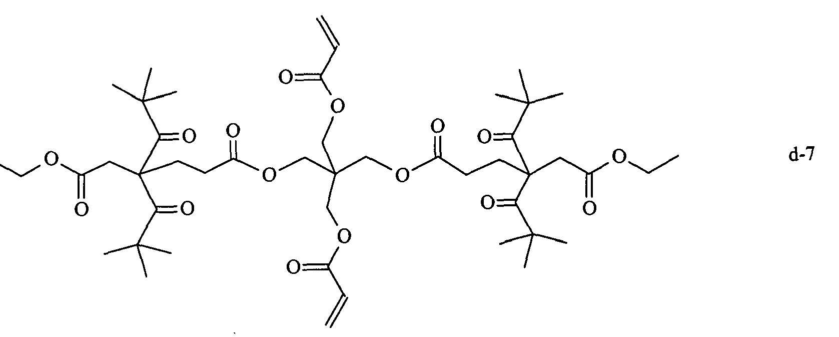

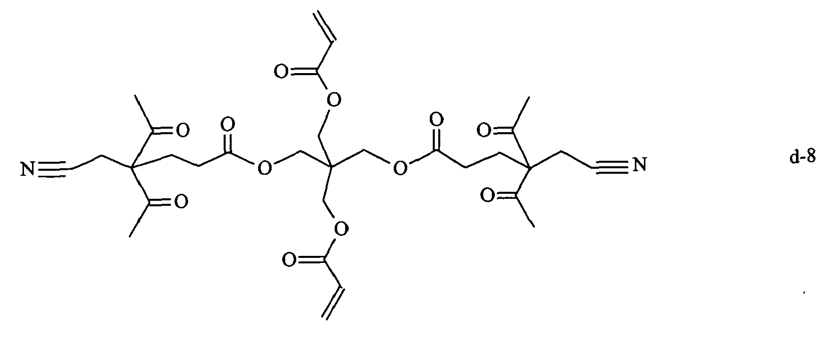

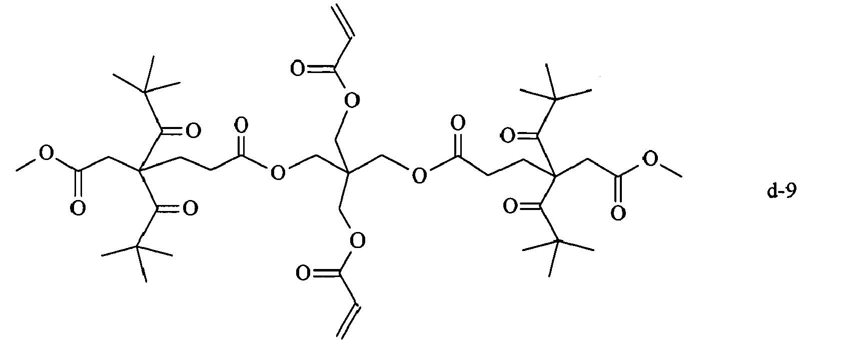

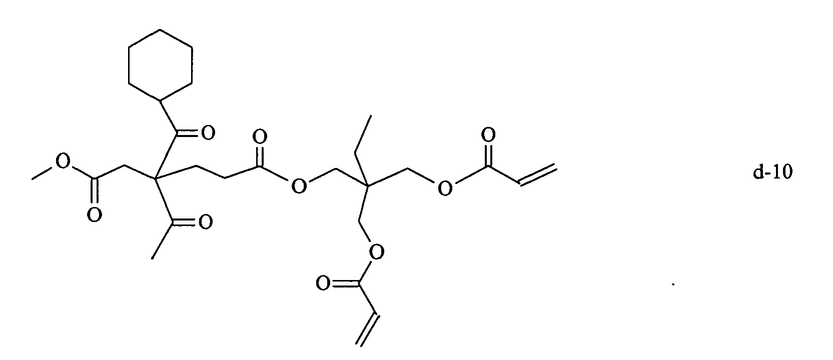

Examples of tri-functional acryloyl group containing compounds, include the compounds represented by the following chemical formula.

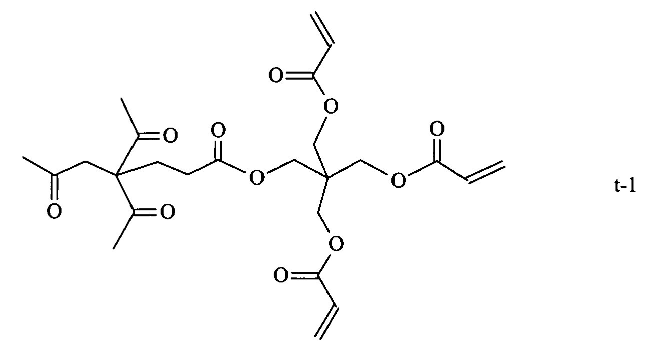

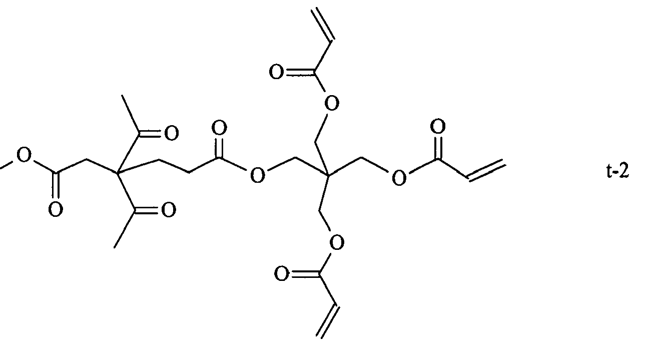

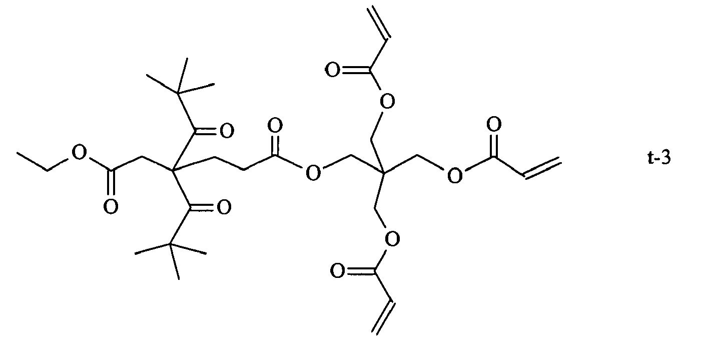

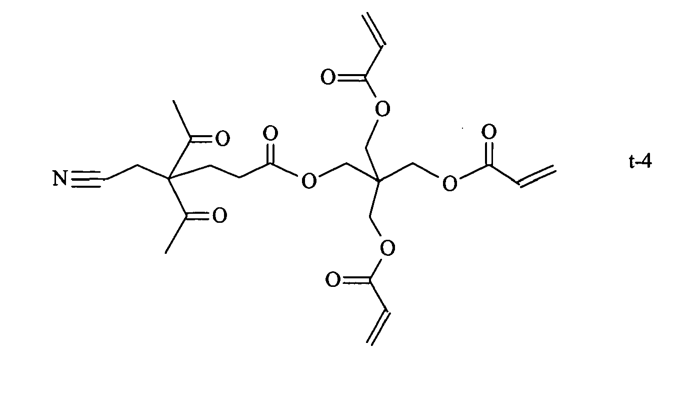

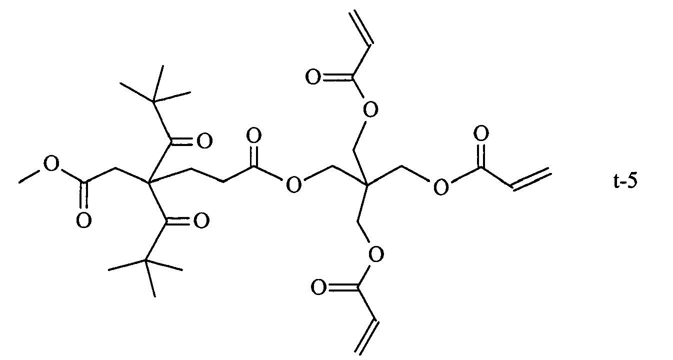

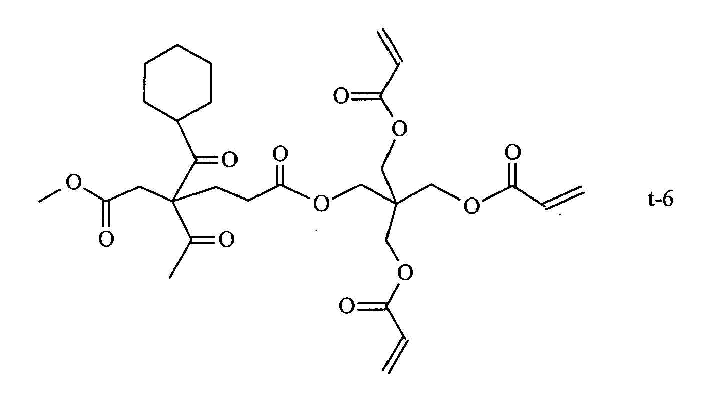

In particular, preferred among these compounds is a photocurable compound of the present invention, which is presented by the following general formula III in light of having superior curability.

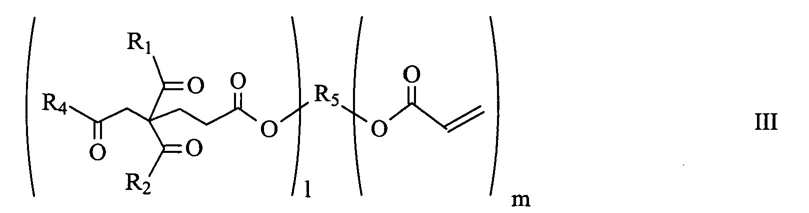

Here, R1 and R2 independently denote an alkyl or cycloalkyl group having a carbon number of 1 to 6, R4 denotes an alkyl or alkoxy group having carbon number of 1 to 8, and R5 denotes a polyvalent aliphatic hydro-carbon group having a carbon number of 2 to 8, 1 means an integer of 1 or 2, and m means an integer of 1 to 3.

Examples of the photocurable compound include the aforementioned chemical formula m-1 to m-5, m-8 to m-18, d-1 to d-3, d-5 to d-7, d-9, d-10, t-1 to t-3, t-5, and t-6.

In the general formula III, the compounds of m=2 or 3, further preferably that of m=2, are preferable from the viewpoint of hardness.

In particular, among these chemical structures represented by the formula III, compounds having a secondary alkyl having carbon number of 3 to 6, tertiary alkyl having carbon number of 4 to 6, or cycloalkyl having carbon number of 5 or 6 as R1 or R2, are preferable from the viewpoints of excellent self-photocurability.

The aforementioned resin (A) has remarkable effects in having day light curability as well as hardness. That is, the composition of the present invention is quite sensitive not only to UV light, but also to sunlight, which contains wavelengths predominantly above 400 nm. Therefore, the resin (A) can be easily cured by sunlight without addition of photoinitiators. Due to this remarkable property, the present composition can be used in the form of a thickly applied layer as coatings. Namely, UV irradiation is not suitable for curing of a thickly applied layer because the resin itself prevents the radiation from being absorbed sufficiently for thorough curing of the resin; on the other hand sunlight is effective for penetrating a thickly applied layer so that the resins are preferably applied for coatings which are usable for a thick layer. The preferable thickness range of the thick layer made of the coatings comprising the resin are, for example, within the range of 500 to 5000 µm. However, in the sunlight curing system, an oxygen atmosphere is not suitable for their curing. Therefore, in order to accelerate the curing speed, sunlight irradiation should be carried out in the absence of oxygen.

The photocurable composition of the invention may comprise the above-mentioned resin (A) alone or may further comprise other photocurable monomers or resins in combination.

Examples of the photocurable monomers include dipropylene glycol diacrylate, trimethyrolpropane-triacrylate, and pentaerythritol tetraacrylate.

Examples of the other photocurable resins include acrylated epoxy resins, acrylated polyurethanes, and acrylated polyesters.

The photocurable composition of the invention may be used as coating, and molded articles because a tack-free and hard cured product can be obtained. Moreover, the composition can be used as printing ink.

Furthermore, the coatings may comprise a proper amount of a colorant such as a dye or a pigment selected from carbon black, titanium white, phthalocyanine, an azo dyestuff or quinacridone, or an inorganic filler such as Si fine particles, mica or calcium carbonate.

Although the photocurable composition exhibits good self curability as mentioned above, furthermore, a known initiator or a known photo-polymerization sensitizer may be added in order to accelerate their photocurability.

The photocurable composition according to the invention as coating can be applied to suitable substrates such as for example paper, polyethylene, polypropylene, polyester, polyvinylidene chloride, aluminum, steel or wood and hardened in air under UV irradiation or under sunlight by a conventional coating method using a roll coater or a knife coater or a conventional printing method such as an offset printing method, a gravure printing method, or a silk screen printing method, to form a film or coating having a thickness of 0.1 to 5000 µm.

Commercially available mercury high-pressure radiators or microwavelength-excited radiators without electrodes may be used for the hardening. Particularly suitable are microwavelength-excited radiators so-called H bulbs. These radiators also emit in a range from 225 to 325 nm in which the composition of the present invention can also absorb.

On the other hand, as mentioned above, the photocurable composition of the present invention can be cured by irradiation with sunlight when the β-diketone group is incorporated in the resin, so that cured thick layers having a thickness of 500 to 5000 µm can be obtained. In the case of using sunlight, in order to exclude oxygen, layers can be cured under an inert gas such as nitrogen, carbon dioxide, or the curable layers can be covered by thin transparent plastic wrap such as polyethylene film. In application as sunlight curable adhesive, which is quite suitable for adhesion of glass articles.
This invention will be further explained by a consideration of the following non-limiting examples which are intended to be purely exemplary of this invention.

### Examples

### EXAMPLE 1

### [Synthesis of the compound (A) represented by the following structure]

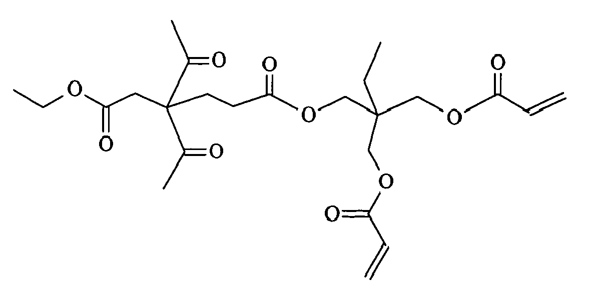

In an 100 ml three necked round-bottom flask equipped with magnetic bar, reflux condenser, thermometer and gas inlet 10.0 g ethyl 3-acetyl-4-oxo-pentanoate, 15.91 g trimethylol propane triacrylate and three drops 1,8-diazabicyclo[5.4.0]undecen-7-ene were mixed. Air was bubbled through the mixture. Exothermic behavior was observed. After 2 hours the mixture has cooled to room-temperature again, 0.1 g hydrochinone mono-methyl ether is added and the mixture stirred at 60 °C for two hours.

The followings are analytical data as to the obtained compound.

| | |
|---|---|
| ¹H-NMR CDCl₃ | 6.40 (4 H; d; ³J_{H-H} = 17.3 Hz; =CH₂); 6.13 (4 H; dd; ³J_{H-H} = 17.3 Hz; -CH=CH₂); 5.87 (4 H; d; ³J_{H-H}= 12.0 Hz; =CH₂); 4.19 (6 H; s; Cq-CH₂-O-C=O)-CH=); 4.11 (2 H; q; ³J_{H-H}= 7.3 Hz; -O-CH₂-CH₃); 2.97 (2 H; s; C_{q}-CH₂-C(=O)O-); 2.38 - 2.32 (4 H; m; >C_{q}-CH₂-CH₂-C(=O)O-); 2.19 (6H; s; C=(O)-CH₃); 1.52 (2 H; m; -CH₂-CH₃); 1.23 (3 H; t; ³J_{H-H} = 7.1 Hz; -O-CH₂-CH₃); 0.95 ppm (3 H; m; CH₂-CH₃) |
| ¹³C-NMR CDCl₃ | 203.9 (s; -C(=O)-CH₃); 171.3 (s; -CH₂-C(=O)-O-CH₂-); 169.9 (s; -(CH₂)₂-C(=O)O-); 164.9 (s; -CH₂-O-C(=O)-CH=CH₂); 130.5 (s; -CH=CH₂); 127.4 (s; -CH=CH₂); 66.9 (s; -CH₂-C(=O)O-CH₂-C_{q}); 63.4 (s; C_{q} -CH₂-O-C(=O)-CH₂-); 60.3 (s; -O-CH₂-CH₃); 40.3 (s; -C(=O)-C_{q}-C(=O)-); 35.6 (s; C_{q}-CH₂-CH₂-C(=O)O-); 28.3 (s; C_{q}CH₂-CH₂-C(=O)O-); 26.0 (s; -C(=O)-CH₃); 25.7 (s; C_{q}-CH₂-C(=O)O-); 22.5 (-CH₂-CH₃); 6.7 ppm (CH₂-CH₃) |

### [Curing Test]

The obtained resin was applied on top of an aluminium sheet so as to be 50 µm film thickness, and was irradiated with a Fusion F300 in one pass at 70 m/min using the H-bulb. This corresponds to an irradiation energy of 0.07 J/cm². Resistance to ethyl methyl ketone was tested by rubbing the surface of the coating with a cotton sheet sucked with ethyl methyl ketone until the surface is reached.
The results are shown in the following table 1.

### Example 2 to 5

The compounds (A) obtained by Example 1 were mixed in accordance with the weight ratio shown in the table 1, then curing tests were done in the same manner of Example 1.

The results are shown in the following table 1.

### Example 6

### Curing with sunlight

The resin obtained by the example 1 is placed in a polyethylene bag of 10 cm x 10 cm x 0.5 cm, the excess of air is removed by suction with vacuum and the bag is sealed. The bag is placed in September at 52 degree latitude on a cloudless day at 3 pm in sunlight. After 7 minutes the bag gets warm, after 15 minutes the bag inside temperature has reached about 45 °C and all liquid material has became solid. The bag is opened after 20 minutes, the solid crosslinked resin sheet is removed and the degree of crosslinking is checked by a gel fraction. Measured gel-fraction is 94 to 95 % by weight, indicating a complete curing of the resin.

### Comparative example 1

In an 250 ml three necked round-bottom flask equipped with magnetic bar, reflux condenser, thermometer and gas inlet, 97.7 g of trimethylol propane triacrylate, 5 g of acetylacetone, and 6.5 of ethyl acetoacetate were mixed, 0.77 g of tri-octylphosphine were added. Then, the mixture was heated, and stirred at 90°C for two hours. When the viscosity of the mixture did not increase any further, heating is discontinued and the product is cooled to room temperature.

As for the obtained resin, curing tests were carried out in the same manner of Example 1 and additionally by using the same application method but curing conditions of 3 passes at 16 m/min belt-speed which means an irradiation energy of 0.6 J / cm² .

The results are shown in the following table 1.

**Table 1**

| | Comp. (A) : TMPTA | Solvent- resistance Ex. 2 | Pencil hardness Ex. 2 | Surface Ex. 2 |
|---|---|---|---|---|
| Example 1 | 1 : 0 | > 200 | 3 H | Tack-free |
| Example 2 | 2 : 1 | > 200 | 3 H | Tack-free |
| Example 3 | 1 : 1 | > 200 | 4 H | Tack-free |
| Example 4 | 1 : 2 | > 200 | 2 H | Tack-free |
| Example 5 | 1 : 3 | > 200 | 2 H | Tack-free |
| Comparative Example 1 | 1 : 0 | 1 at 0.07 J/cm² | - | Liquid |
| | | >75 at 0.6 J/cm² | 3H | Tack-free |

As shown by the results, the compositions of the present invention, show higher curability, which can be recognized by hardness of films such as solvent resistance.

## Claims

1. A photocurable composition containing a resin (A) having an acryloyl group and a chemical structural element represented by the following formula I, 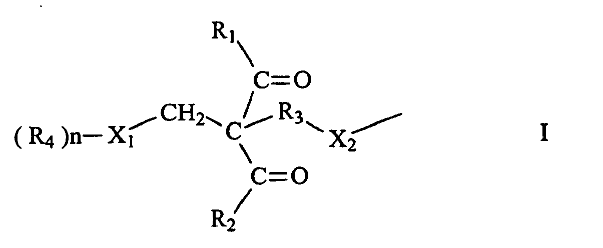 wherein R1 and R2 independently denote an alkyl or cycloalkyl group having a carbon number of 1 to 6, X1 denotes carbonyl group or cyano group, R4 denotes an alkyl or alkoxy group having carbon number of 1 to 8, n denotes 1 when X1 is carbonyl, and 0 when X1 is cyano group, R3 denotes an alkylene group having a carbon number of 1 to 4, and X2 denotes an electron attracting group.

2. A photocurable composition according to claim 1, wherein the resin (A) has an acryloyl group and a chemical structure element represented by the following formula II, 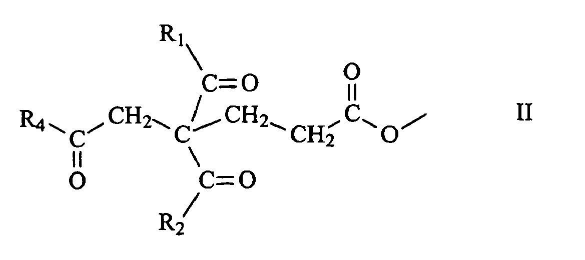 wherein R1 and R2 independently denote an alkyl or cycloalkyl group having a carbon number of 1 to 6, R4 denotes an alkyl or alkoxy group having carbon number of 1 to 8.

3. A photocurable composition according to claims 1 to 4, wherein the resin (A) has a molecular weight (Mn) of 300 to 20,000.

4. A photocurable composition according to claims 1 to 4, wherein the acyl groups represented by the following formula A1 and A2 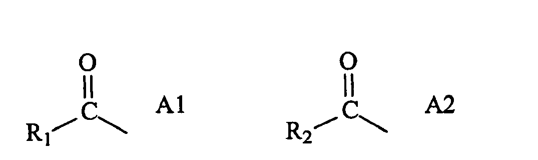 wherein R1 and R2 denotes an alkyl group having a carbon number of 1 to 6, exist at the rate of between 1·10⁻⁵ mole per gram and 1·10⁻² mole per gram based on the total amount of the composition.

5. A photocurable composition according to claim 1, wherein the composition further contains one or several acrylic monomers.

6. A photocurable compound, which is represented by the following formula III, 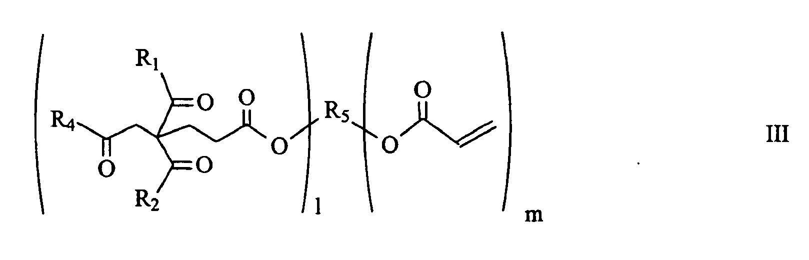 wherein R1 and R2 independently denote an alkyl or cycloalkyl group having a carbon number of 1 to 6, R4 denotes an alkyl or alkoxy group having carbon number of 1 to 8, R5 denotes a polyvalent aliphatic hydro-carbon group having a carbon number of 2 to 8, 1 means an integer of 1 or 2, and m means an integer of 1 to 3.

## Patentansprüche

1. Photohärtbare Zusammensetzung enthaltend ein Harz (A) mit einer Acryloylgruppe und einem chemischen Strukturelement, verkörpert durch die folgende Formel I, 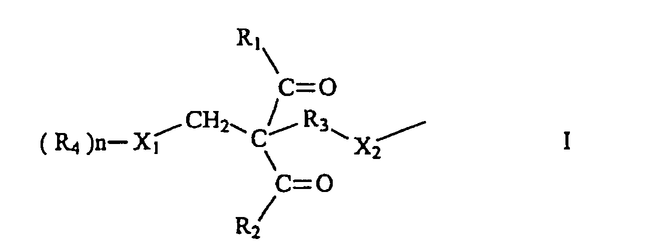 worin R₁ und R₂ unabhängig eine Alkyl- oder Cycloalkylgruppe mit einer Kohlenstoffanzahl von 1 bis 6 bezeichnen, X₁ eine Carbonylgruppe oder Cyanogruppe bezeichnet, R₄ eine Alkyl- oder Alkoxygruppe mit einer Kohlenstoffanzahl von 1 bis 8 bezeichnet, n 1 bezeichnet wenn X₁ Carbonyl ist, und 0 wenn X₁ eine Cyanogruppe ist, R₃ eine Alkylengruppe mit einer Kohlenstoffanzahl von 1 bis 4 bezeichnet, und X₂ eine elektronenanziehende Gruppe bezeichnet.

2. Photohärtbare Zusammensetzung gemäß Anspruch 1, worin das Harz (A) eine Acryloylgruppe und ein chemisches Strukturelement, verkörpert durch die folgende Formel II hat, 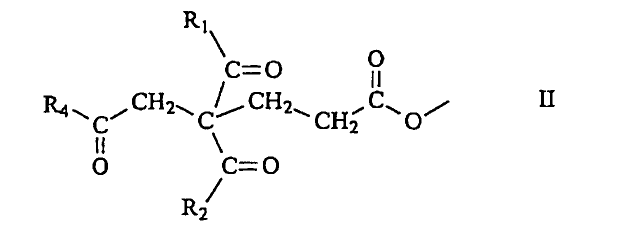 worin R₁ und R₂ unabhängig eine Alkyl- oder Cycloalkylgruppe mit einer Kohlenstoffanzahl von 1 bis 6 bezeichnen, R₄ eine Alkyl- oder Alkoxygruppe mit einer Kohlenstoffanzahl von 1 bis 8 bezeichnet.

3. Photohärtbare Zusammensetzung gemäß Ansprüchen 1 bis 4, wobei das Harz (A) ein Molekulargewicht (Mn) von 300 bis 20.000 besitzt.

4. Photohärtbare Zusammensetzung gemäß Ansprüchen 1 bis 4, wobei die durch die folgenden Formeln A1 und A2 verkörperten Acylgruppen 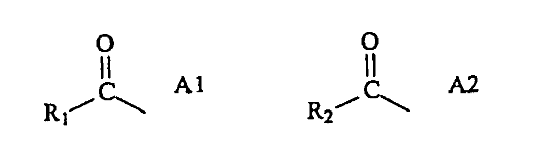 worin R₁ und R₂ eine Alkylgruppe mit einer Kohlenstoffanzahl von 1 bis 6 bezeichnen, in einer Rate von zwischen 1-10⁻⁵ Mol pro Gramm und 1·10⁻² Mol pro Gramm, bezogen auf die Gesamtmenge der Zusammensetzung, vorliegen.

5. Photohärtbare Zusammensetzung gemäß Anspruch 1, wobei die Zusammensetzung des Weiteren ein oder mehrere Acrylmonomer(e) enthält.

6. Photohärtbare Verbindung, die durch die folgende Formel III verkörpert wird, 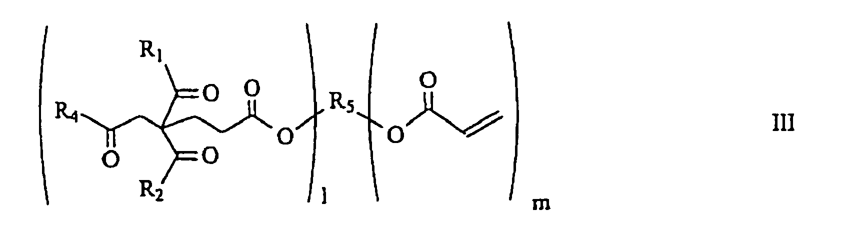 worin R₁ und R₂ unabhängig eine Alkyl- oder Cycloalkylgruppe mit einer Kohlenstoffanzahl von 1 bis 6 bezeichnen, R₄ eine Alkyl- oder Alkoxygruppe mit einer Kohlenstoffanzahl von 1 bis 8 bezeichnet, R₅ eine polyvalente aliphatische Kohlenwasserstoffgruppe mit einer Kohlenstoffanzahl von 2 bis 8 bezeichnet, 1 eine ganze Zahl von 1 oder 2 bedeutet und m eine ganze Zahl von 1 bis 3 bedeutet.

## Revendications

1. Composition photodurcissable contenant une résine (A) ayant un groupe acryloyle et un élément de structure chimique représenté par la formule (I) suivante 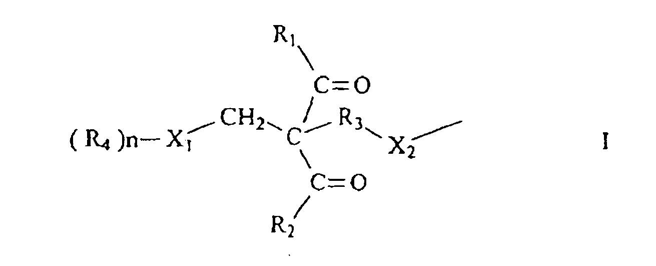 dans laquelle R₁ et R₂ représentent indépendamment un groupe alkyle ou cycloalkyle ayant un nombre de carbones de 1 à 6, X₁ représente un groupe carbonyle ou cyano, R₄ représente un groupe alkyle ou alcoxy ayant un nombre de carbones de 1 à 8, n est égal à 1 lorsque X₁ est un groupe carbonyle, et est égal à 0 lorsque X₁ est un groupe cyano, R₃ représente un groupe alkylène ayant un nombre de carbones de 1 à 4, et X₂ représente un groupe attracteur d'électrons.

2. Composition photodurcissable selon la revendication 1,
dans laquelle la résine (A) a un groupe acryloyle et un élément de structure chimique représenté par la formule (II) suivante 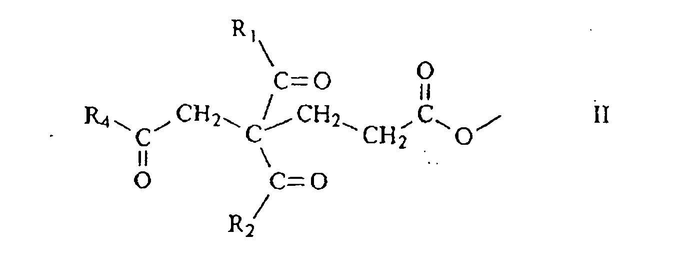 dans laquelle R₁ et R₂ représentent indépendamment un groupe alkyle ou cycloalkyle ayant un nombre de carbones de 1 à 6, R₄ représente un groupe alkyle ou alcoxy ayant un nombre de carbones de 1 à 8.

3. Composition photodurcissable selon les revendication 1 à 4, dans laquelle la résine (A) a un poids moléculaire (Mn) de 300 à 20.000.

4. Composition photodurcissable selon les revendications 1 à 4, dans laquelle les groupes acyles représentés par les formules A1 et A2 suivantes : 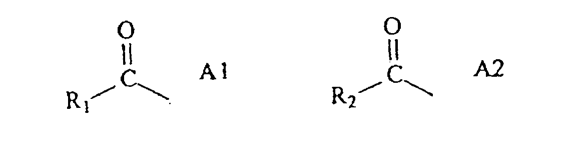 dans lesquelles R₁ et R₂ représentent un groupe alkyle ayant un nombre de carbones de 1 à 6, sont présents dans une proportion comprise entre 1 x 10⁻⁵ mole par gramme et 1 x 10⁻² mole par gramme, par rapport à la quantité totale dans la composition.

5. Composition photodurcissable selon la revendication 1, dans laquelle la composition contient aussi un ou plusieurs monomères acryliques.

6. Composé photodurcissable qui est représenté par la formule (III) suivante : 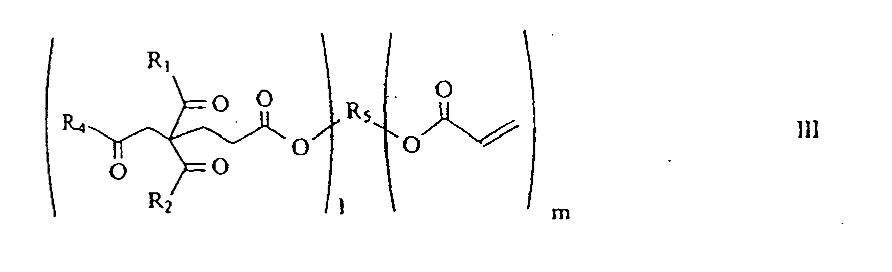 dans laquelle R₁ et R₂ représentent indépendamment un groupe alkyle ou cycloalkyle ayant un nombre de carbones de 1 à 6, R₄ représente un groupe alkyle ou alcoxy ayant un nombre de carbones de 1 à 8, R₅ représente un groupe hydrocarboné aliphatique polyvalent ayant un nombre de carbones de 2 à 8, 1 est un nombre entier égal à 1 ou à 2, et m est un nombre entier égal à 1, 2 ou 3.
